# EUROPEAN PATENT SPECIFICATION

(11) **EP 1 674 062 B1**
(45) Date of publication and mention of the grant of the patent: **21.09.2016**
(21) Application number: 04030649.0
(22) Date of filing: 23.12.2004
(51) Int. Cl.: A61F 11/14, H04R 25/00

(54) **Personal monitoring system for a user and method for monitoring a user**
Persönliche Überwachungssystem für einen Benützer und eine Methode zur Benützerüberwachung
Système personnel de contrôle d'un utilisateur et méthode pour surveiller un utilisateur

(43) Date of publication of application: 28.06.2006
(73) Proprietor: Sonova AG, 8712 Stäfa (CH)
(72) Inventor: Berg, Christian, 8713 Uerikon (CH); Bächler, Herbert, 8706 Meilenerg (CH)
(74) Representative: Schwan Schorer & Partner mbB

(56) References cited:
- EP-A- 1 448 014
- WO-A-01/82798
- US-A- 5 721 783
- US-A1- 2002 080 979
- US-B1- 6 661 901

## Description

The invention relates to a personal monitoring system for a user comprising a sensor for sensing an ambient parameter of the ambience around the user, an earpiece for being worn at least in part in the user's ear canal including an acoustic output transducer for providing sound to the user's ear canal, an evaluation unit and a compliance control unit for providing the user with acoustic information regarding compliance of the sensed parameter with regulations. The invention further relates to a corresponding monitoring method.

US 6,661,901 B1 relates to an active hearing protection device comprising an earplug with a customized shell wherein, in addition to the outer microphone for sensing unattenuated ambient sound, the audio signal processing unit and the speaker adapted to provide sound signals to the user's ear canal, an inner microphone is provided which is adapted to sense the sound level prevailing within the user's ear canal when wearing the hearing protection earplug, i.e. the inner microphone is provided for in-situ measurement of the actual sound exposure experienced by the user's ear. The earplug includes a noise exposure dosimeter function, according to which a stationary or semi-stationary noise dose is obtained by A-weighing the signal provided by the inner microphone and accumulating the squared values. Peak values are obtained by C-weighing the signal of the inner microphone and saving the peak values. The noise dose and the peak values are compared to predetermined limits and, if the limits are exceeded, an audible information is given via the speaker included within the earplug, to the user in form of warning signals or synthetic speech.

US 5,721,783 relates to a system comprising an earpiece to be worn in the user's ear canal which is adapted for wireless communication with a remote processor unit which may be worn under the user's clothing. The earpiece comprises a microphone for sensing ambient sounds, an audio signal processing unit and a speaker for providing sound signals to the user's ear canal. The remote processor unit may be connected to sensors and peripheral devices which may provide information regarding body temperature, heart pulse or blood sugar level of the user by measuring the corresponding parameters, wherein the information obtained thereby is provided to the user in the form of audio signals via the speaker of the earpiece, such as by synthetic speech. In addition, external sensors communicating with the remote processor unit may be used for obtaining user location information or for detecting nuclear, biological and chemical weapons or for detecting metal parts. The obtained information is provided to the user by appropriate verbal warnings or distinctive alerting tones generated by the remote processor unit and sent to the earpiece for perception as audio signals by the user.

US 2002/0080979 A1 relates to an active hearing protection system comprising two earpieces, wherein an analyzing circuit including an evaluation unit and a compliance control unit is placed within the earpieces.

It is an object of the invention to provide for a personal monitoring system for a user regarding parameters of the ambience to which the user is exposed, wherein the system should be particularly well adapted to the individual needs of the user and wherein the user should be provided with efficient feedback from the system regarding the monitoring results. It is a further object of the invention to provide for a corresponding personal monitoring method.

These objects are achieved by a monitoring system as defined in claim 1 and a monitoring method as defined in claim 21.

The invention is beneficial in that, by individually implementing an individually defined regulation for the sensed parameter(s) into an evaluation unit, highly individual monitoring, i.e. monitoring specifically adapted to the individual needs of the user, is enabled. By providing the user with acoustic information regarding the compliance of the sensed parameter(s) with the implemented individual regulations via an acoustic output transducer provided in an earpiece worn by the user, feedback regarding the results of the monitoring is provided to the user in an efficient and reliable manner, without other persons being disturbed by sound signals provided by the monitoring system.

Preferably, the earpiece is a hearing protection device providing for a mechanical acoustic attenuation of at least 10 dB averaged over the audible frequency range when worn by the user. To this end, the earpiece may comprise a customized shell, i.e. a shell having an outer surface individually shaped according to the measured inner shape of the user's outer ear and ear canal. Preferably, the shell has an elasticity from shore D85 to shore D65 and may be made of polyamide. Preferably, the shell is produced by an additive layer-by-layer build-up process, such as layer-by-layer laser sintering of a polyamide powder. The sensor is a microphone located at the outer side of the earpiece for sensing the ambient sound exposure experienced by the user. The earpiece may further include an audio signal processing unit for processing audio signals provided by the microphone as input to the output transducer, whereby an active hearing protection device can be realized. In this respect, the audio signal processing unit may be designed to modify the amplification and frequency shaping of the processed audio signals according to the level and frequencies of the sensed ambient sound. In order to monitor the sound exposure experienced by the user, the evaluation unit is adapted to determine separate sound levels for a plurality of frequency bands over time, wherein the individually defined regulation includes for each of the frequency bands a sound level threshold value and an alarm limit for an accumulated time period during which the respective sound level threshold value is exceeded. The individually defined regulation may depend on the acoustic attenuation performance of the earpiece.

Further, the individually defined regulation may include for each of the sound frequency bands a recovery limit for the respective accumulated time period during which the respective sound level threshold value is not exceeded. The individually defined regulation includes for each of the frequency bands a plurality of sound level ranges and associated alarm limits for the respective accumulated time period during which the sensed sound level falls within the respective sound level range. The individually defined regulation may include for each of the sound level ranges a recovery limit for the respective accumulated time period during which the sensed sound level does not fall within the respective sound level range. If recovery limits are defined, the evaluation unit judges that the individually defined regulation is not complied with if at least one of the alarm time interval limits is exceed without the respective recovery time interval limit having been reached.

If the earpiece is designed as an active hearing protection device, the amplification of the processed audio signals may be modified according to the judgement made by the evaluation unit.

The evaluation unit may be adapted to continuously classify the ambient sound regarding at least one parameter, such as pleasant/unpleasant impression to the user, in addition to sound level, by frequency analysis.

In addition to the sound exposure by the user, the system may sense and monitor one of the following parameters: air temperature, humidity, air pressure, thermal radiation, X-ray intensity, radioactivity, chemical air composition and air contamination. If the system senses and monitors an internal body parameter, this parameter may be body temperature, blood pressure and/or heart pulse rate. Preferably, in this case the individually defined regulation regarding this parameter is defined depending on the medication of the user. In addition, the individually defined regulation regarding this parameter may be defined depending on at least one sensed ambient parameter or on at least one additionally sensed internal body parameter. For example, the upper limit of the heart pulse may be defined depending on the sensed blood pressure of the user and depending on the sensed air temperature and air pressure.

In general, the sensor preferably will be integrated within the earpiece. This will be possible for most ambient parameters, while the sensing of internal body parameters in some cases may require an external sensor worn by the user. However, also for sensing ambient parameters it is possible to use an external sensor, for example, a sensor permanently installed at a facility. Preferably communication between the earpiece and the external sensor is wireless. Preferably, the earpiece includes means for producing speech signals, alarm signals or pre-defined sounds as the acoustic feedback signals provided to the user, wherein explicit speech instructions to the user depending on the judgement made by the evaluation unit are preferred in order to provide for a highly reliable feedback to the user. The individually defined regulation may be implemented in the system from a remote data input device via a detachable wired or wireless data connection with a data input interface provided at the earpiece.

Preferably, the evaluation unit and the compliance control unit are formed by a digital signal processing unit which is integrated within the earpiece.

In the following, examples of the invention will be illustrated by reference to the attached drawings.
Fig. 1 shows a schematic view of an embodiment of a monitoring system which is not covered by the invention;
Fig. 2 shows a view like Fig. 1 of a first embodiment of the invention; and
Fig. 3 shows a view like Fig. 1 of a second embodiment of the invention.

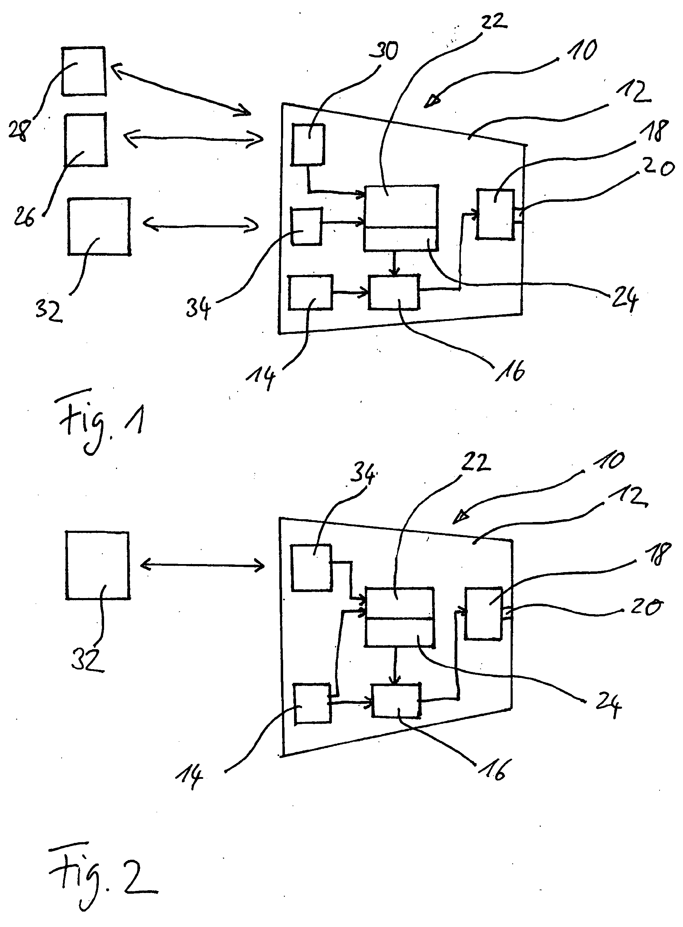

Fig. 1 relates to a personal monitoring system for a user, which is not covered by the invention and which is designed as a hearing protection earplug 10 comprising a shell 12 which is adapted to be worn at least in part in a user's ear canal, i.e. at least a distal portion of the shell is to be inserted into the outer part of the user's ear canal in order to provide for an acoustic attenuation of at least 10 dB averaged over the audible frequency range when the earplug is worn by the user, in order to protect the user from excessive levels of ambient sound. The earplug may comprise an acoustic filter for adjusting the desired total acoustic attenuation or for adjusting the frequency dependent acoustic attenuation.

The shell preferably is a hard shell having an elasticity from shore D85 to D65 and preferably is made of polyamide. In order to achieve optimized fit of the shell within the user's outer ear and ear canal, the shell preferably has an outer surface individually shaped according to the measured shape of the user's outer ear and ear canal, i.e. the shell preferably has an individually customized outer shape. The shape of the user's outer ear and ear canal may be determined by direct three-dimensional scanning of the ear canal and the concha or by producing an impression of the ear canal and the concha which subsequently undergoes scanning. The scanning process may be carried out optically, preferably by laser scanning.

The digital data obtained by the scanning process is then used to create the hard shell by an additive or incremental layer-by-layer build up process. Such processes are also known as "rapid prototyping". A preferred additive build-up process is a layer-by-layer laser sintering process of powder material, preferably polyamide powder. Such processes are also known as "selective laser sintering" (SLS). The basic principle therein is the repeated deposition of a thin layer of material on a surface, with the desired sectional shape then being stabilized, i.e. hardened, by laser action. An overview regarding such processes can be found, for example, in US 2003/0133583 A1 or US 6,533,062 B1.

The earplug 10 of Fig. 1 is provided with an active hearing protection function, i.e. the shell 12 comprises a microphone 14. for converting ambient sound into input audio signals, an audio signal processing unit 16 for processing the input audio signals into output audio signals and an acoustic output transducer (speaker) 18 which converts the output audio signal into sound provided at a sound outlet opening 20 at the distal end of the shell 12. Theses components are provided for allowing selected sound perception, such as speech signals, by the user even when wearing the earplug 10 in a noisy environment.

Further, the earplug 10 comprises an evaluation unit 22 and a compliance control unit 24. The evaluation unit 22 communicates with external sensors 26 and 28 via an interface 30. The interface 30 may be adapted for wireless communication with the sensors 26 and 28 or may serve to connect the sensors 26, 28 by a corresponding wire connection to the evaluation unit 22. The sensors 26, 28 may be designed for sensing an internal body parameter of the user, such as body temperature, blood pressure or heart pulse rate, and/or for sensing an ambient parameter of the ambient around the user, such as temperature, humidity, air pressure, thermal radiation, X-ray intensity, radio activity, chemical air composition and air contamination. The sensors 26, 28 may be adapted to be worn by the user or to be permanently installed at a facility.

The evaluation unit 22 is designed for monitoring values of the parameters sensed by the sensors 26, 28 over time and comparing them to individually defined regulations for these parameters. The evaluation unit 22 is further designed for continuously judging whether the sensed values of theses parameters comply with the respective individual regulation or not. The respective individual regulations for the sensed parameters are individually implemented into the evaluation unit 22 from an external data input device 32 via an interface 34 provided at the earplug 10. Data transfer between the data input device 32 and the interface 34 may occur in a wireless manner or via a detachable wire data connection.

The compliance control unit 24 communicates with the evaluation unit 22 and is designed for providing acoustic signals to the user's ear via the speaker 18 in order to provide the user with acoustic information regarding the present compliance of the sensed parameters with the corresponding implemented individual regulations depending on the judgement made by the evaluation unit 22. To this end, the compliance control unit 24 is connected to the audio signal processing unit 16. The acoustic feedback provided to the user may be provided in the form of synthetic speech signals, alarm signals or pre-defined sounds. Preferably, the acoustic feedback is provided in the form of explicit speech instructions to the user depending on the judgement made by the evaluation unit 22.

The individual regulation implemented in the evaluation unit 22 could be, for example an individual prescription from a physician (for example, the prescription to avoid excessive physical activity if temperature, altitude and/or humidity are above certain limits) or working environment regulations (for example, the regulation to work only a given number of hours per day if the temperature is above a given limit, the regulation to refrain from working if radioactivity or air contamination is above a given limit, etc.). The individual regulation may be defined depending on the medication of the user. The individual regulation for a body parameter may depend on the value of at least one other sensed parameter, such as a further body parameter or an ambient parameter. This also applies to the case where the individual regulation relates to an ambient parameter.

Fig. 2 relates to an embodiment in which a sensor is integrated within the earplug 10. In the example shown in Fig. 2 the sensor is a microphone 14, which serves two functions: On the one hand, the microphone 14 is provided, similarly to the embodiment of Fig. 1, for providing the hearing protection earplug 10 with an active hearing protection function via the audio signal processing unit 16 and the speaker 18; on the other hand, the microphone 14 serves to continuously measure the ambient sound exposure of the user wearing the earplug 10. To this end, the microphone 14 is not only connected with the audio signal processing unit 16 but also with the evaluation unit 22. As in the embodiment of Fig. 1, the evaluation unit 22 in addition is connected to the interface 34 for allowing an individual regulation regarding the sound levels sensed by the microphone 14 to be implemented via the external data input device 32.

The embodiment of Fig. 2 may serve to provide for a hearing protection ear plug 10 with a noise exposure control function, i.e. a dosimeter function. To this end, the evaluation unit 22 may be designed to determine separate sound levels for a plurality of frequency bands over time. Further, the evaluation unit 22 may be designed to continuously classify the ambient sound regarding one additional parameter, such as pleasant/unpleasant impression to the user, by frequency analysis, wherein the individually defined regulation includes a condition for this additional parameter and this additional parameter is used in the judgement made by the avaluation unit 22.

The individually defined regulation includes for each of the frequency bands a sound level threshold value and an alarm limit for an accumulated time period during which the respective sound level threshold value is exceeded. The individually defined regulation may depend on the acoustic attenuation performance of the earplug 10.

Further, the individually defined regulation may include for each of the sound frequency bands a recovery limit for the respective accumulated time period during which the respective sound level threshold value is not exceeded. The individually defined regulation may further include for each of the frequency bands a plurality of sound level ranges and associated alarm limits for the respective accumulated time period during which the sensed sound level falls within the respective sound level range. Also in this case, the individually defined regulation may include for each of the sound level ranges a recovery limit for the respective accumulated time period during which the sensed sound level does not fall within the respective sound level range. If recovery limits are defined, the evaluation unit 22 judges that the individually defined regulation is not complied with if at least one of the alarm time interval limits is exceed without the respective recovery time interval limit having been reached.

The amplification of the audio signals processed by the audio signal processing unit 16 may be modified according to the judgement made by the evaluation unit 22.

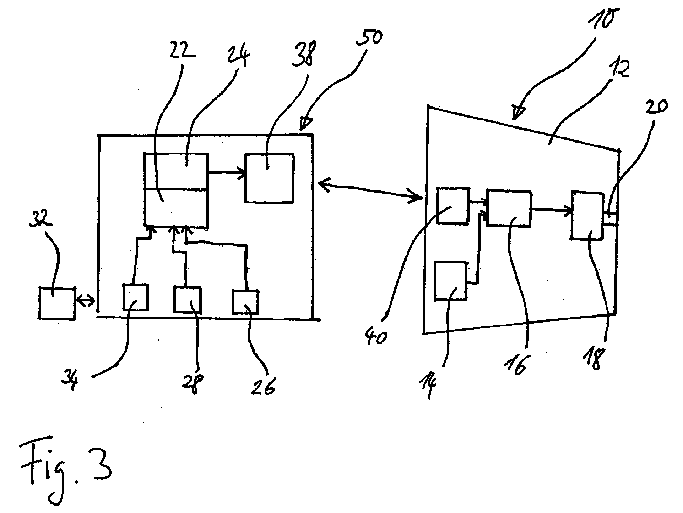

In Fig. 3 a modified embodiment is shown, wherein the evaluation unit 22 and the compliance control unit 24 are not integrated within the earplug 10 but rather are included in an external unit 50 which also includes the sensors 26, 28 of Fig. 1. The external unit 50 is provided with an interface 38 for communicating with an interface 40 provided at the earplug 10 in order to provide for acoustic feedback to the user via the speaker 18 of the earplug 10. Preferably, the communication between the earplug 10 and the external unit 50 occurs via a wireless connection; however, alternatively also a wire connection would be possible which preferably would be detachable.

The external unit 50 is provided with the interface 34 for allowing the individual regulation being implemented into the evaluation unit 22 from the external data input device 32.

Preferably, the external unit 50 is designed to be worn by the user.

## Claims

1. A personal monitoring system for a user, comprising: a sensor (14, 26, 28) for sensing an ambient parameter of the ambient around said user, an earpiece (10) for being worn at least in part in the ear canal of said user including an acoustic output transducer (18) for providing sound to the user's ear canal, an evaluation unit (22) communicating with said sensor, means (32, 34) for implementing a regulation for said sensed parameter into said evaluation unit, said evaluation unit being adapted for monitoring sensed values of said parameter over time and comparing them to said regulation for said sensed parameter and being adapted for continuously judging whether said sensed values of said parameter comply with said regulation or not, and a compliance control unit (24) communicating with said evaluation unit and with said output transducer for providing acoustic signals to the user's ear via said output transducer for providing the user with acoustic information regarding the present compliance of said sensed parameter with said implemented regulation depending on the judgement made by said evaluation unit, wherein said ambient parameter sensor is a microphone (14) located at the outer side of said earpiece for sensing the ambient sound exposure experienced by said user, **characterized in that** said regulation is an individually defined and individually implemented regulation which includes for each frequency band a sound level threshold value and an alarm limit for an accumulated time period during which the respective sound level threshold value is exceeded.

2. The system of claim 1, wherein said earpiece is a hearing protection earplug (10) providing for a mechanical acoustic attenuation of at least 10 dB averaged over the audible frequency range when worn by said user.

3. The system of claim 2, wherein said earpiece comprises a shell (12) having an outer surface individually shaped according to the measured inner shape of the user's outer ear and ear canal.

4. The system of claim 3, wherein said shell (12) is a hard shell with an elasticity of from shore D85 to shore D65.

5. The system of one of the preceding claims, wherein said earpiece (10) includes an audio signal processing unit (16) for processing audio signals provided by said microphone (14) as input to said output transducer (18).

6. The system of claim 5, wherein said audio signal processing unit (16) is designed to modify the amplification and the frequency shaping of the processed audio signals according to the level and the frequencies of the sensed ambient sound.

7. The system of one of the preceding claims, wherein said evaluation unit (22) is adapted to determine separate sound levels for a plurality of frequency bands over time.

8. The system of one of the preceding claims, wherein said evaluation unit (22) is adapted to continuously classify the ambient sound regarding at least one parameter, such as pleasant/unpleasant impression to the user, in addition to sound level by frequency analysis.

9. The system of one of the preceding claims, wherein an additional sensor (26, 28) adapted to sense one of the following ambient parameters is provided: air temperature, humidity, air pressure, thermal radiation, X-ray intensity, radioactivity, chemical air composition and air contamination.

10. The system of one of the preceding claims, wherein a sensor (26, 28) adapted to sense an internal body parameter selected from the group consisting of body temperature, blood pressure and heart pulse rate is provided.

11. The system of one of the preceding claims, wherein said microphone (14) is integrated within said earpiece.

12. The system of claim 9, wherein said additional sensor is an external sensor (26, 28) and wherein means (30, 38, 40) are provided for establishing a wired or wireless communication connection between said earpiece (10) and said external sensor.

13. The system of claim 12, wherein said external sensor (26,28) is adapted to be worn by said user.

14. The system of claim 12, wherein said external sensor (26, 28) is adapted to be permanently installed at a facility.

15. The system of one of the preceding claims, wherein said earpiece (10) includes means (16, 24) for producing speech signals, alarm signals or pre-defined sounds as said acoustic signals to be provided to the user.

16. The system of claim 15, wherein said acoustic signals include explicit speech instructions to the user depending on the judgement made by said evaluation unit (22).

17. The system of one of the preceding claims, wherein said means for individually implementing said individually defined regulation include a remote data input device (32) and a data input interface (34) provided at the earpiece (10).

18. The system of claim 17, wherein said data input interface (34) is adapted for establishing a detachable wired data connection with said remote data input device (32).

19. The system of claim 17, wherein said data input interface (34) is adapted for establishing a wireless data connection with said remote data input device (32).

20. The system of one of the preceding claims, wherein said evaluation unit (22) and said compliance control unit (24) are formed by a digital signal processing unit, with said earpiece comprising said digital signal processing unit.

21. A method for monitoring a user, comprising:
sensing an ambient parameter of the ambient around said user;
implementing a regulation for said sensed parameter into an evaluation unit (22);
monitoring, by said evaluation unit, sensed values of said parameter over time and comparing them to said regulation for said sensed parameter;
continuously judging, by said evaluation unit, whether said sensed values of said parameter comply with said regulation or not;
providing acoustic signals to the user's ear via an output transducer (18) located in an earpiece (10) worn at least in part in the ear canal of said user for providing the user with acoustic feedback regarding the present compliance of said sensed parameter with said implemented regulation depending on the judgement made by said evaluation unit, wherein said sensed ambient parameter is the ambient sound exposure experienced by said user, with the ambient sound being converted into audio signals by a microphone (14) provided at the earpiece (10), **characterized in that** said regulation is individually defined and individually implemented and includes for each frequency band a sound level threshold value and an alarm limit for an accumulated time period during which the respective sound level threshold value is exceeded.

22. The method of claim 21, further comprising processing said audio signals as input to said output transducer (18).

23. The method of one of claims 21 or 22, further comprising modifying the amplification of the processed audio signals according to the level and the frequencies of the sensed ambient sound.

24. The method of claim 21 to 23, further comprising determining, by said evaluation unit (22), separate sound levels for a plurality of frequency bands over time.

25. The method of one of claims 21 to 24, further comprising continuously classifying, by said evaluation unit (22), the ambient sound regarding at least one parameter, such as pleasant/unpleasant impression to the user, in addition to sound level, by frequency analysis, wherein said individually defined regulation includes a condition for said additional parameter and said additional parameter is used in the judgement made by the evaluation unit.

26. The method of one of claims 21 to 25, wherein said individually defined regulation includes for each of said sound frequency bands a recovery limit for the respective accumulated time period during which the respective sound level threshold value is not exceeded.

27. The method of claim 26, wherein said individually defined regulation includes for each of said frequency bands a plurality of sound level ranges and associated alarm limits for the respective accumulated time period during which the sensed sound level falls within the respective sound level range.

28. The method of claim 27, wherein said individually defined regulation includes for each of said sound level ranges a recovery limit for the respective accumulated time period during which the sensed sound level does not fall within the respective sound level range.

29. The method of one of claims 26 to 28, wherein said evaluation unit (22) judges that said individually defined regulation is not complied with if at least one of the alarm limits is exceeded without the respective recovery limit having been reached.

30. The method of one of claims 21 to 29, wherein said earpiece (10) is worn in a manner as to provide for a mechanical acoustic attenuation of at least 10 dB averaged over the audible frequency range.

31. The method of claim 30, wherein said individually defined regulation depends on the acoustic attenuation performance of said earpiece (10).

32. The method of one of claims 21 to 31, further comprising modifying the amplification of the processed audio signals according to the judgement made by said evaluation unit (22).

33. The method of one of claims 21 to 32, wherein said microphone (14) is integrated within said earpiece.

34. The method of claim 21, further comprising establishing a wired or wireless communication connection between said earpiece (10) and an external sensor (26, 28) for sensing an ambient parameter of the ambient around said user.

35. The method of claim 34, wherein said external sensor (26, 28) is worn by said user.

36. The method of claim 34, wherein said external sensor (26, 28) is permanently installed at a facility.

37. The method of one of claims 21 to 36, wherein said acoustic signals provided to the user's ear include speech signals.

38. The method of claim 37, wherein said acoustic signals provided to the user's ear include explicit instructions to the user depending on the judgement made by said evaluation unit (22).

39. The method of claim 34, wherein said sensed ambient parameter is one of air temperature, humidity, air pressure, thermal radiation, X-ray intensity, radioactivity, chemical air composition and air contamination.

40. The method of claim 21, wherein an internal body parameter selected from body temperature, blood pressure and heart pulse rate is sensed.

41. The method of one of claims 21 to 40, wherein said individually defined regulation is defined depending on the medication of said user.

42. The method of one of claims 21 to 41, wherein said individually defined regulation is defined depending on at least one sensed ambient parameter.

43. The method of one of claims 21 to 42, wherein said individually defined regulation is defined depending on at least one sensed internal body parameter.

44. The method of one of claims 21 to 43, further comprising establishing a detachable wired data connection or a wireless data connection with a remote data input device (32) for individually implementing said individually defined regulation.

## Patentansprüche

1. Persönliches Überwachungssystem für einen Nutzer mit: einem Sensor (14, 26, 28) zum Erfassen eines Umgebungsparameters einer den Nutzer umgebenden Umgebung, einem Ohrstück (10) zum Tragen mindestens zum Teil im Gehörgang des Nutzers, mit einem akustischen Ausgangswandler (18) zum Versorgen des Gehörgangs des Nutzers mit Schall, einer Bewertungseinheit (22), die mit dem Sensor kommuniziert, Mitteln (32, 34) zum Implementieren einer Vorschrift für den erfassten Parameter in der Bewertungseinheit, die ausgebildet ist, um erfasste Werte des Parameters über die Zeit zu überwachen und sie mit der Vorschrift für den erfassten Parameter zu vergleichen, und die ausgebildet ist, um kontinuierlich zu beurteilen, ob die erfassten Werte des Parameters die Vorschrift erfüllen oder nicht, und einer Erfüllungssteuereinheit (24), die mit der Bewertungseinheit und dem Ausgangswandler kommuniziert, um dem Ohr des Nutzers akustische Signale über den Ausgangswandler zuzuführen, um den Nutzer mit akustischer Information zu versorgen hinsichtlich der gegenwärtigen Erfüllung der implementierten Vorschrift durch den erfassten Parameter, in Abhängigkeit von der durch die Bewertungseinheit vorgenommenen Beurteilung, wobei es sich bei dem Umgebungsparametersensor um ein Mikrofon (14) handelt, welches an der Außenseite des Ohrstücks angeordnet ist, um die von dem Nutzer empfangene Umgebungsschallexposition zu erfassen, **dadurch gekennzeichnet, dass** es sich bei der Vorschrift um eine individuell definierte und individuell implementierte Vorschrift handelt, welche für jedes Frequenzband einen Schallpegelschwellwert und eine Alarmgrenze für einen akkumulierten Zeitraum, während welchem der entsprechende Schallpegelschwellwert überschritten wird, beinhaltet.

2. System gemäß Anspruch 1, wobei es sich bei dem Ohrstück um einen Gehörschutzohrstöpsel (10) handelt, der für eine mechanische Schalldämpfung von mindestens 10 dB, gemittelt über den hörbaren Frequenzbereich, sorgt, wenn er von dem Nutzer getragen wird.

3. System gemäß Anspruch 2, wobei das Ohrstück eine Schale (12) aufweist, die eine Außenfläche aufweist, die gemäß der gemessenen Innenform des Außenohrs und Gehörgangs des Nutzers individuell geformt ist.

4. System gemäß Anspruch 3, wobei es sich bei der Schale (12) um eine harte Schale mit einer Elastizität zwischen Shore D85 und Shore D65 handelt.

5. System gemäß irgendeinem der vorhergehenden Ansprüche, wobei das Ohrstück (10) eine Audiosignalverarbeitungseinheit (16) zum Verarbeiten von von dem Mikrofon (14) als Eingangssignal für den Ausgangswandler (18) bereitgestellten Audiosignalen beinhaltet.

6. System gemäß Anspruch 5, wobei die Audiosignalverarbeitungseinheit (16) ausgelegt ist, um die Verstärkung und die Frequenzformung des verarbeiteten Audiosignals gemäß dem Pegel und den Frequenzen des erfassten Umgebungsschalls zu modifizieren.

7. System gemäß einem der vorhergehenden Ansprüche, wobei die Bewertungseinheit (22) ausgebildet ist, um getrennte Schallpegel für eine Mehrzahl von Frequenzbändern über die Zeit zu bestimmen.

8. System gemäß einem der vorhergehenden Ansprüche, wobei die Bewertungseinheit (22) ausgebildet ist, um den Umgebungsschall hinsichtlich mindestens eines Parameters, wie beispielsweise angenehmer/unangenehmer Eindruck für den Nutzer, zusätzlich zu dem Schallpegel mittels Frequenzanalyse kontinuierlich zu klassifizieren.

9. System gemäß einem der vorhergehenden Ansprüche, wobei ein zusätzlicher Sensor (26, 28) vorgesehen ist, der ausgebildet ist, um mindestens einen der folgenden Umgebungsparameter zu erfassen: Lufttemperatur, Feuchtigkeit, Luftdruck, thermische Strahlung, Röntgenstrahlenintensität, Radioaktivität, chemische Luftzusammensetzung und Luftverschmutzung.

10. System gemäß einem der vorhergehenden Ansprüche, wobei ein Sensor (26, 28) vorgesehen ist, der ausgebildet ist, um einen inneren Körperparameter zu erfassen, der aus der aus Körpertemperatur, Blutdruck und Herzpulsrate bestehenden Gruppe ausgewählt ist.

11. System gemäß einem der vorhergehenden Ansprüche, wobei das Mikrofon (14) innerhalb des Ohrstücks integriert ist.

12. System gemäß Anspruch 9, wobei es sich bei dem zusätzlichen Sensor um einen externen Sensor (26, 28) handelt und wobei Mittel (30, 38, 40) vorgesehen sind, um eine drahtgebundene oder drahtlose Kommunikationsverbindung zwischen dem Ohrstück (10) und dem externen Sensor aufzubauen.

13. System gemäß Anspruch 12, wobei der externe Sensor (26, 28) ausgebildet ist, um von dem Nutzer getragen zu werden.

14. System gemäß Anspruch 12, wobei der externe Sensor (26, 28) ausgebildet ist, um permanent in einer Einrichtung installiert zu sein.

15. System gemäß einem der vorhergehenden Ansprüche, wobei das Ohrstück (10) Mittel (16, 24) aufweist, um Sprachsignale, Alarmsignale oder vordefinierte Schallereignisse als die dem Nutzer bereitzustellenden akustischen Signale zu erzeugen.

16. System gemäß Anspruch 15, wobei die akustischen Signale explizite Sprachanweisungen an den Nutzer in Abhängigkeit von der von der Bewertungseinheit (22) vorgenommenen Beurteilung aufweisen.

17. System gemäß einem der vorhergehenden Ansprüche, wobei die Mittel zum individuellen Implementieren der individuell definierten Vorschrift ein Ferndateneingabegerät (32) und eine an dem Ohrstück (10) vorgesehene Dateneingabeschnittstelle (34) aufweisen.

18. System gemäß Anspruch 17, wobei die Dateneingabeschnittstelle (34) ausgebildet ist, um eine lösbare drahtgebundene Datenverbindung mit dem Ferndateneingabegerät (32) aufzubauen.

19. System gemäß Anspruch 17, wobei die Dateneingabeschnittstelle (34) ausgebildet ist, um eine drahtlose Datenverbindung mit dem Ferndateneingabegerät (32) aufzubauen.

20. System gemäß einem der vorhergehenden Ansprüche, wobei die Bewertungseinheit (22) und die Erfüllungssteuereinheit (24) durch eine digitale Signalverarbeitungseinheit gebildet sind, wobei das Ohrstück die digitale Signalverarbeitungseinheit aufweist.

21. Verfahren zum Überwachen eines Nutzers, wobei:
ein Umgebungsparameter der den Nutzer umgebenden Umgebung erfasst wird;
eine Vorschrift für den erfassten Parameter in einer Bewertungseinheit (22) implementiert wird;
erfasste Werte des Parameters mittels der Bewertungseinheit über die Zeit überwacht werden und mit der Vorschrift für den erfassten Parameter verglichen werden,
mittels der Bewertungseinheit kontinuierlich beurteilt wird, ob die erfassten Werte des Parameters die Vorschrift erfüllen oder nicht;
dem Ohr des Nutzers mittels eines Ausgangswandlers (18), der in einem Ohrstück angeordnet ist, welches mindestens zum Teil in dem Gehörgang des Nutzers getragen wird, akustische Signale zugeführt werden, um den Nutzer mit akustischer Rückmeldung bezüglich der gegenwärtigen Erfüllung der implementierten Vorschrift durch den erfassten Parameter in Abhängigkeit von der von der Bewertungseinheit vorgenommenen Beurteilung zu versorgen, wobei es sich bei dem erfassten Umgebungsparameter um die von dem Nutzer erfahrene Umgebungsschallexposition handelt, wobei der Umgebungsschall mittels eines an dem Ohrstück (10) vorgesehenen Mikrofons (14) in Audiosignale umgewandelt wird, **dadurch gekennzeichnet, dass** die Vorschrift individuell definiert und individuell implementiert wird und für jedes Frequenzband einen Schallpegelschwellwert und eine Alarmgrenze für eine akkumulierte Zeitdauer, während welcher der entsprechende Schallpegelschwellwert überschritten wird, beinhaltet.

22. Verfahren gemäß Anspruch 21, wobei ferner die Audiosignale als Eingangssignal für den Ausgangswandler (18) verarbeitet werden.

23. Verfahren gemäß irgendeinem der Ansprüche 21 oder 22, wobei ferner die Verstärkung der verarbeiteten Audiosignale gemäß dem Pegel und den Frequenzen des erfassten Umgebungsschalls modifiziert wird.

24. Verfahren gemäß irgendeinem der Ansprüche 21 bis 23, wobei ferner mittels der Bewertungseinheit (22) getrennte Schallpegel für eine Mehrzahl von Frequenzbändern über die Zeit bestimmt werden.

25. Verfahren gemäß irgendeinem der Ansprüche 21 bis 24, wobei ferner mittels der Bewertungseinheit der Umgebungsschall hinsichtlich mindestens eines Parameters, wie beispielsweise einem angenehmen/unangenehmen Eindruck für den Nutzer, zusätzlich zu dem Schallpegel mittels Frequenzanalyse kontinuierlich klassifiziert wird, wobei die individuell definierte Vorschrift eine Bedingung für den zusätzlichen Parameter beinhaltet und der zusätzliche Parameter in der von der Bewertungseinheit vorgenommenen Beurteilung verwendet wird.

26. Verfahren gemäß einem der Ansprüche 21 bis 25, wobei die individuell definierte Vorschrift für jedes der Schallfrequenzbänder eine Erholungsgrenze für die entsprechende akkumulierte Zeitdauer beinhaltet, während welcher der entsprechende Schallpegelschwellwert nicht überschritten wird.

27. Verfahren gemäß Anspruch 26, wobei die individuell festgelegte Vorschrift für jedes der Frequenzbänder eine Mehrzahl von Schallpegelbereichen und zugeordneten Alarmgrenzen für die entsprechende akkumulierte Zeitdauer beinhaltet, während welcher der erfasste Schallpegel in den entsprechenden Schallpegelbereich fällt.

28. Verfahren gemäß Anspruch 27, wobei die individuell definierte Vorschrift für jeden der Schallpegelbereiche eine Erholungsgrenze für die entsprechend akkumulierte Zeitdauer aufweist, während welcher der erfasste Schallpegel nicht in den entsprechenden Schallpegelbereich fällt.

29. Verfahren gemäß einem der Ansprüche 26 bis 28, wobei die Bewertungseinheit (22) urteilt, dass die individuell definierte Vorschrift nicht erfüllt wird, falls mindestens eine der Alarmgrenzen überschritten wird, ohne dass die entsprechende Erholungsgrenze erreicht wurde.

30. Verfahren gemäß einem der Ansprüche 21 bis 29, wobei das Ohrstück (10) in einer Weise getragen wird, um für eine mechanische akustische Dämpfung von mindestens 10 dB, gemittelt über den hörbaren Frequenzbereich, zu sorgen.

31. Verfahren gemäß Anspruch 30, wobei die individuell definierte Vorschrift von der akustischen Dämpfungsleistungsfähigkeit des Ohrstücks (10) abhängt.

32. Verfahren gemäß einem der Ansprüche 21 bis 31, wobei ferner die Verstärkung des verarbeiteten Audiosignals gemäß der von der Bewertungseinheit (22) vorgenommenen Beurteilung modifiziert wird.

33. Verfahren gemäß einem der Ansprüche 21 bis 32, wobei das Mikrofon (14) in das Ohrstück integriert ist.

34. Verfahren gemäß Anspruch 21, wobei ferner eine drahtgebundene oder drahtlose Verbindung zwischen dem Ohrstück (10) und einem externen Sensor (26, 28) zum Erfassen eines Umgebungsparameters der den Nutzer umgebenden Umgebung aufgebaut wird.

35. Verfahren gemäß Anspruch 34, wobei der externe Sensor (26, 28) von dem Nutzer getragen wird.

36. Verfahren gemäß Anspruch 34, wobei der externe Sensor (26, 28) permanent in einer Einrichtung installiert ist.

37. Verfahren gemäß einem der Ansprüche 21 bis 36, wobei die dem Ohr des Nutzers zugeführten akustischen Signale Sprachsignale beinhalten.

38. Verfahren gemäß Anspruch 37, wobei die dem Ohr des Nutzers zugeführten Audiosignale explizite Anweisungen an den Nutzer in Abhängigkeit von der von der Bewertungseinheit (22) vorgenommenen Beurteilung aufweisen.

39. Verfahren gemäß Anspruch 34, wobei es sich bei dem erfassten Umgebungsparameter um Lufttemperatur, Feuchtigkeit, Luftdruck, thermische Strahlung, Röntgenstrahlungsintensität, Radioaktivität, chemische Luftzusammensetzung und/oder Luftverschmutzung handelt.

40. Verfahren gemäß Anspruch 21, wobei ein innerer Körperparameter erfasst wird, bei dem es sich um Körpertemperatur, Blutdruck oder Herzpulsrate handelt.

41. Verfahren gemäß einem der Ansprüche 21 bis 40, wobei die individuell definierte Vorschrift in Abhängigkeit von der Medikamentierung des Nutzers definiert wird.

42. Verfahren gemäß einem der Ansprüche 21 bis 41, wobei die individuell definierte Vorschrift in Abhängigkeit von mindestens einem erfassten Umgebungsparameter definiert wird.

43. Verfahren gemäß einem der Ansprüche 21 bis 42, wobei die individuell definierte Vorschrift in Abhängigkeit von mindestens einem erfassten inneren Körperparameter definiert wird.

44. Verfahren gemäß einem der vorhergehenden Ansprüche 21 bis 43, wobei ferner eine lösbare drahtgebundene Datenverbindung oder eine drahtlose Datenverbindung mit einem Ferndateneingabegerät (32) aufgebaut wird, um die individuell definierte Vorschrift individuell zu implementieren.

## Revendications

1. Système de contrôle personnel pour un utilisateur, comprenant : un capteur (14, 26, 28) pour détecter un paramètre ambiant du milieu ambiant autour de l'utilisateur, un écouteur (10) à porter au moins en partie dans le canal auriculaire de l'utilisateur et comprenant un transducteur de sortie acoustique (18) pour fournir un son au canal auriculaire de l'utilisateur, une unité d'évaluation (22) communiquant avec le capteur, des moyens (32, 34) pour mettre en oeuvre une régulation pour le paramètre détecté dans l'unité d'évaluation, laquelle unité d'évaluation est conçue pour contrôler des valeurs détectées du paramètre dans le temps et les comparer à la régulation pour le paramètre détecté, et est conçue pour juger en continu si les valeurs détectées du paramètre sont conformes ou non à la régulation, et une unité de commande de conformité (24) communiquant avec l'unité d'évaluation et avec le transducteur de sortie pour émettre des signaux acoustiques vers l'oreille de l'utilisateur via le transducteur de sortie afin de fournir à l'utilisateur des informations acoustiques concernant la présente conformité du paramètre détecté à la régulation mise en oeuvre en fonction du jugement réalisé par l'unité d'évaluation, dans lequel le détecteur de paramètre ambiant est un microphone (14) situé sur le côté externe de l'écouteur afin de détecter l'exposition au son ambiant que ressent l'utilisateur, **caractérisé en ce que** la régulation est une régulation définie individuellement et mise en oeuvre individuellement qui comprend pour chaque bande de fréquence une valeur seuil de niveau sonore et une limite d'alarme pour une période de temps accumulée pendant laquelle la valeur seuil de niveau sonore respective est dépassée.

2. Système selon la revendication 1, dans lequel l'écouteur est une oreillette de protection auditive (10) assurant une atténuation acoustique mécanique d'au moins 10 dB en moyenne sur la plage de fréquence audible lorsqu'elle est portée par l'utilisateur.

3. Système selon la revendication 2, dans lequel l'écouteur comprend une coque (12) ayant une surface externe formée individuellement en fonction de la forme interne mesurée de l'oreille externe et du canal auriculaire de l'utilisateur.

4. Système selon la revendication 3, dans lequel la coque (12) est une coque rigide ayant une élasticité allant de D85 shore à D65 shore.

5. Système selon l'une des revendications précédentes, dans lequel l'écouteur (10) comprend une unité de traitement de signal audio (16) pour traiter des signaux audio fournis par le microphone (14) comme entrée vers le transducteur de sortie (18).

6. Système selon la revendication 5, dans lequel l'unité de traitement de signal audio (16) est conçue pour modifier l'amplification et la mise en forme de fréquence des signaux audio traités en fonction du niveau et des fréquences du son ambiant détecté.

7. Système selon l'une des revendications précédentes, dans lequel l'unité d'évaluation (22) est conçue pour déterminer des niveaux sonores distincts pour plusieurs bandes de fréquence dans le temps.

8. Système selon l'une des revendications précédentes, dans lequel l'unité d'évaluation (22) est conçue pour classer en continu le son ambiant selon au moins un paramètre, comme une impression agréable/désagréable pour l'utilisateur, en plus du niveau sonore par analyse de fréquence.

9. Système selon l'une des revendications précédentes, dans lequel est fourni un capteur additionnel (26, 28) conçu pour détecter un des paramètres ambiants suivants : température de l'air, humidité, pression de l'air, rayonnement thermique, intensité de rayons X, radioactivité, composition chimique de l'air et contamination de l'air.

10. Système selon l'une des revendications précédentes, dans lequel est fourni un capteur (26, 28) conçu pour détecter un paramètre corporel interne choisi dans le groupe comprenant la température du corps, la pression sanguine et le pouls.

11. Système selon l'une des revendications précédentes, dans lequel le microphone (14) est intégré dans l'écouteur.

12. Système selon la revendication 9, dans lequel le capteur additionnel est un capteur externe (26, 28) et des moyens (30, 38, 40) sont utilisés pour établir une connexion de communication filaire ou sans fil entre l'écouteur (10) et le capteur externe.

13. Système selon la revendication 12, dans lequel le capteur externe (26, 28) est conçu pour être porté par l'utilisateur.

14. Système selon la revendication 12, dans lequel le capteur externe (26, 28) est conçu pour être installé à demeure au niveau d'un local.

15. Système selon l'une des revendications précédentes, dans lequel l'écouteur (10) comprend un moyen (16, 24) pour produire des signaux vocaux, des signaux d'alarme ou des sons prédéfinis comme les signaux acoustiques devant être fournis à l'utilisateur.

16. Système selon la revendication 15, dans lequel les signaux acoustiques comprennent des instructions vocales explicites pour l'utilisateur en fonction du jugement effectué par l'unité d'évaluation (22).

17. Système selon l'une des revendications précédentes, dans lequel les moyens pour mettre en oeuvre individuellement la régulation définie individuellement comprennent un dispositif d'entrée de données distant (32) et une interface d'entrée de données (34) au niveau de l'écouteur (10).

18. Système selon la revendication 17, dans lequel l'interface d'entrée de données (34) est conçue pour établir une connexion de données filaire détachable avec le dispositif d'entrée de données distant (32).

19. Système selon la revendication 17, dans lequel l'interface d'entrée de données (34) est conçue pour établir une connexion de données sans fil avec le dispositif d'entrée de données distant (32).

20. Système selon l'une des revendications précédentes, dans lequel l'unité d'évaluation (22) et l'unité de commande de conformité (24) sont formées par une unité de traitement de signaux numériques, l'écouteur comprenant ladite unité de traitement de signaux numériques.

21. Procédé de contrôle d'un utilisateur consistant à :
- détecter un paramètre ambiant du milieu ambiant autour de l'utilisateur ;
- mettre en oeuvre une régulation pour le paramètre détecté dans une unité d'évaluation (22) ;
- contrôler par l'unité d'évaluation les valeurs détectées du paramètre dans le temps et les comparer à la régulation pour le paramètre détecté ;
- juger en continu par l'unité d'évaluation si les valeurs détectées du paramètre sont conformes ou non à la régulation ;
- émettre des signaux acoustiques vers l'oreille de l'utilisateur via un transducteur de sortie (18) situé dans un écouteur (10) porté au moins en partie dans le canal auriculaire de l'utilisateur afin de fournir à l'utilisateur un retour acoustique concernant la conformité présente du paramètre détecté à la régulation mise en oeuvre en fonction du jugement effectué par l'unité d'évaluation, le paramètre ambiant détecté étant l'exposition au son ambiant que ressent l'utilisateur, le son ambiant étant converti en signaux audio par un microphone (14) disposé au niveau de l'écouteur (10), **caractérisé en ce que** la régulation est définie individuellement et mise en oeuvre individuellement et comprend pour chaque bande de fréquence une valeur seuil de niveau sonore et une limite d'alarme pour une période de temps accumulée pendant laquelle la valeur seuil de niveau sonore respective est dépassée.

22. Procédé selon la revendication 21, consistant en outre à traiter les signaux audio comme une entrée vers le transducteur de sortie (18).

23. Procédé selon l'une des revendications 21 ou 22, consistant en outre à modifier l'amplification des signaux audio traités en fonction du niveau et des fréquences du son ambiant détecté.

24. Procédé selon les revendications 21 à 23, consistant en outre à déterminer par l'unité d'évaluation (22) des niveaux sonores distincts pour plusieurs bandes de fréquence dans le temps.

25. Procédé selon l'une des revendications 21 à 24, consistant en outre à classer en continu par l'unité d'évaluation (22) le son ambiant selon au moins un paramètre, comme une impression agréable/désagréable pour l'utilisateur, en plus du niveau sonore par analyse de fréquence, la régulation définie individuellement comprenant une condition pour le paramètre additionnel et le paramètre additionnel étant utilisé dans le jugement effectué par l'unité d'évaluation.

26. Procédé selon l'une des revendications 21 à 25, dans lequel la régulation définie individuellement comprend pour chacune des bandes de fréquence sonores une limite de récupération pour la période de temps accumulée respective pendant laquelle la valeur seuil de niveau sonore respective n'est pas dépassée.

27. Procédé selon la revendication 26, dans lequel la régulation définie individuellement comprend pour chacune des bandes de fréquence plusieurs plages de niveau sonore et des limites d'alarme associées pour la période de temps accumulée respective pendant laquelle le niveau sonore détecté se trouve dans la plage de niveau sonore respective.

28. Procédé selon la revendication 27, dans lequel la régulation définie individuellement comprend pour chacune des plages de niveau sonore une limite de récupération pour la période de temps accumulée respective pendant laquelle le niveau sonore détecté ne se trouve pas dans la plage de niveau sonore respective.

29. Procédé selon l'une des revendications 26 à 28, dans lequel l'unité d'évaluation (22) juge que la régulation définie individuellement n'est pas respectée si au moins l'une des limites d'alarme est dépassée sans que la limite de récupération respective ait été atteinte.

30. Procédé selon l'une des revendications 21 à 29, dans lequel l'écouteur (10) est porté de manière à fournir une atténuation acoustique mécanique d'au moins 10 dB en moyenne sur la plage de fréquence audible.

31. Procédé selon la revendication 30, dans lequel la régulation définie individuellement dépend de la performance d'atténuation acoustique de l'écouteur (10).

32. Procédé selon l'une des revendications 21 à 31, consistant en outre à modifier l'amplification des signaux audio traités en fonction du jugement effectué par l'unité d'évaluation (22).

33. Procédé selon l'une des revendications 21 à 32, dans lequel le microphone (14) est intégré dans l'écouteur.

34. Procédé selon la revendication 21, consistant en outre à établir une connexion de communication filaire ou sans fil entre l'écouteur (10) et un capteur externe (26, 28) afin de détecter un paramètre ambiant du milieu ambiant autour de l'utilisateur.

35. Procédé selon la revendication 34, dans lequel le capteur externe (26, 28) est porté par l'utilisateur.

36. Procédé selon la revendication 34, dans lequel le capteur externe (26, 28) est installé à demeure au niveau d'un local.

37. Procédé selon l'une des revendications 21 à 36, dans lequel les signaux acoustiques fournis à l'oreille de l'utilisateur comprennent des signaux vocaux.

38. Procédé selon la revendication 37, dans lequel les signaux acoustiques fournis à l'oreille de l'utilisateur comprennent des instructions explicites pour l'utilisateur en fonction du jugement effectué par l'unité d'évaluation (22).

39. Procédé selon la revendication 34, dans lequel le paramètre ambiant détecté est l'un des paramètres suivants : température de l'air, humidité, pression de l'air, rayonnement thermique, intensité de rayons X, radioactivité, composition chimique de l'air et contamination de l'air.

40. Procédé selon la revendication 21, dans lequel on détecte un paramètre corporel interne choisi parmi la température du corps, la pression sanguine et le pouls.

41. Procédé selon l'une des revendications 21 à 40, dans lequel la régulation définie individuellement est définie en fonction du traitement médicamenteux de l'utilisateur.

42. Procédé selon l'une des revendications 21 à 41, dans lequel la régulation définie individuellement est définie en fonction d'au moins un paramètre ambiant détecté.

43. Procédé selon l'une des revendications 21 à 42, dans lequel la régulation définie individuellement est définie en fonction d'au moins un paramètre corporel interne détecté.

44. Procédé selon l'une des revendications 21 à 43, consistant en outre à établir une connexion de données filaire détachable ou une connexion de données sans fil avec un dispositif d'entrée de données distant (32) afin de mettre en oeuvre individuellement la régulation définie individuellement.
